# EUROPÄISCHE PATENTSCHRIFT

(11) **EP 1 275 736 B1**
(45) Veröffentlichungstag und Bekanntmachung des Hinweises auf die Patenterteilung: **15.06.2005**
(21) Anmeldenummer: 01116865.5
(22) Anmeldetag: 10.07.2001
(51) Int. Cl.: C12Q 1/68

(54) **Verfahren zur Identifizierung von Leserastermutationen**
Method for detection of frameshift mutations
Procédé pour la détection de mutations du cadre de lecture

(43) Veröffentlichungstag der Anmeldung: 15.01.2003
(73) Patentinhaber: ApoGene Biotechnologie GmbH & Co KG, 86567 Hilgertshausen-Larezhausen (DE)
(72) Erfinder: Brem, Gottfried, Prof. Dr. Dr., 86567 Hilgertshausen (DE); Czerny, Thomas, Institut für Tierzucht und Genetik, 1210 Wien (AT)
(74) Vertreter: Becker Kurig Straus

(56) Entgegenhaltungen:
- EP-A- 0 872 560
- US-A- 5 876 940

## Beschreibung

Die vorliegende Erfindung betrifft ein einfaches und schnelles Verfahren zur Identifizierung von Mutationen in einem Gen von Interesse, die eine Veränderung des davon abgeleiteten Proteins nach sich ziehen. Die vorliegende Erfindung betrifft insbesondere die Verwendung dieses Verfahrens bei der Durchmusterung von Populationen zur Isolatierung von Mutanten sowie zur Früherkennung der Neigung der Individuen Krankheiten zu entwickeln.

Grundlage einer jeden evolutionären Entwicklung ist eine natürliche Mutationsrate, durch die das Genom spontane Veränderungen erfährt. Obwohl ein Großteil dieser Mutationen keinerlei essentielle Funktionen betreffen, kann ein bestimmter Prozentsatz dennoch erhebliche Konsequenzen nach sich ziehen, wie beispielsweise das Entstehen oder die Neigung zur Entwicklung von Erkrankungen, die an die Nachkommen weitergegeben werden (Erbkrankheiten). Sind derartige Mutationen rezessiv, so entstehen für das betroffene Individuum vorerst keinerlei Nachteile, da das nicht betroffene Allel bzw. dessen Produkt die biologische Aufgabe weiter erfüllen wird. In jeder neuen Generation einer Population kommen daher rezessive Mutationen im allgemeinen ohne einhergehende Veränderung des Phänotyps vor. Erst bei einer Kreuzung mit einem zweiten heterozygoten Individuum, das eine Mutation im selben Gen aufweist, sind negative Auswirkungen an den Nachkommen zu erwarten.

Induzierte Mutationen, wie beispielsweise durch gezielten Einsatz von Chemikalien und/oder Strahlung, haben sich in der genetischen Analyse von biologischen wie auch medizinischen Fragestellungen als äußerst hilfreich erwiesen. Wird ein Elternteil mutagenen Reagenzien ausgesetzt, so entstehen in der folgenden F1 Generation heterozygote Nachkommen, die Mutationsträger sind, jedoch meist keinen veränderten Phänotyp aufweisen. Erst ein kompliziertes Kreuzungsschema, das bis in die F3 Generation weitergeht ermöglicht die Sichtbarmachung der Phänotypen dieser rezessiven Mutationen in homozygoten Individuen. Aufgrund der komplexen und zeitaufwendigen Verfahren des Standes der Technik zur Bestimmung derartiger Mutationen besteht ein Bedarf nach einfacheren Verfahren.

In der EP 0 872 560 ist bereits ein Verfahren zur Erfassung von Unsinns-Mutationen beschrieben, bei dem mittels homologer Rekombination ein Konstrukt hergestellt wird, das das zu untersuchende Gen stromabwärts eines Promotors und, im gleichen Leseraster mit dem zu untersuchenden Gen, ein sogenanntes Reportergen enthält. Bei Expression des Leserasters wird ein Fusionsprotein gebildet, das die Reportergenfunktion enthält. Dieses kann mittels phänotypischer Eigenschaften nachgewiesen werden, wie der Widerstandsfähigkeit gegenüber Antibiotika, Enzymen, die mittels entsprechender Verfahrens erfasst werden können, oder der Fähigkeit der das Konstrukt enthaltenen Zellen auf bestimmten Nährböden zu wachsen.

Ein Nachteil dieses Verfahrens besteht jedoch darin, daß in einem ersten Schritt auf die erfolgreiche Rekombination des zu testenden Genfragments mit dem Vektor gescreent werden muß. Erst in einem zweiten Schritt kann dann das Vorhandensein der Reporterfunktion nachgewiesen werden. Ein weiteres Problem dieses Verfahrens besteht in der Erfassung heterozygoter Mutationen, die aufgrund des Nachweises phänotypischer Eigenschaften, wie der von dem Genprodukt des Reportergens abgeleiteten Enzymaktivität, großen Variationen unterworfen sind, die eine verlässliche Aussage letztendlich nicht zulassen. Gemäß dem offenbarten Verfahren werden Mutationen nur durch die Abwesenheit positiver Kolonien bemerkbar. Heterozygot mutierte Individuen zeigen daher im Vergleich mit einem wildtyp Individuum lediglich einen zweifachen Unterschied in der Zahl der positiven Kolonien, wobei statistisch bedingte Variationen die Aussagekraft des Verfahrens für das Vorhandensein von heterozygoten Mutationen beträchtlich beeinträchtigen.

Eine Aufgabe der vorliegenden Erfindung besteht daher darin ein schnelles Verfahren zur Bestimmung von Mutationen in einem Gen von Interesse bereitzustellen, das zuverlässig und einfach durchzuführen sein soll. Das Verfahren soll sich insbesondere für eine Durchmusterung von Populationen auf Mutation eignen, bei der eine Vielzahl von Individuen schnell untersucht werden.

Die vorstehend aufgeführte Aufgabe wird durch ein Verfahren zum Nachweis von Mutationen in einem Gen, bei denen der native Leseraster zerstört wird, gelöst, bei dem ein Konstrukt hergestellt wird, das eine Nucleotidsequenz enthält, die mindestens einem Teil der codierenden Region eines Gens von Interesse entspricht, sowie eine ein Toxin codierende Nucleotidsequenz, das stromabwärts der Nucleotidsequenz des Gens von Interesse mit diesem im Leseraster davon verknüpft ist. Das Konstrukt wird nach Einbringen in eine geeignete Wirtszelle darin exprimiert.

In den Figuren,
- Fig. 1: zeigt schematisch ein Vektorkonstrukt zum Einsatz in der vorliegenden Erfindung,
- Fig. 2: zeigt schematisch den Aufbau einer Platte bei der Transformation.

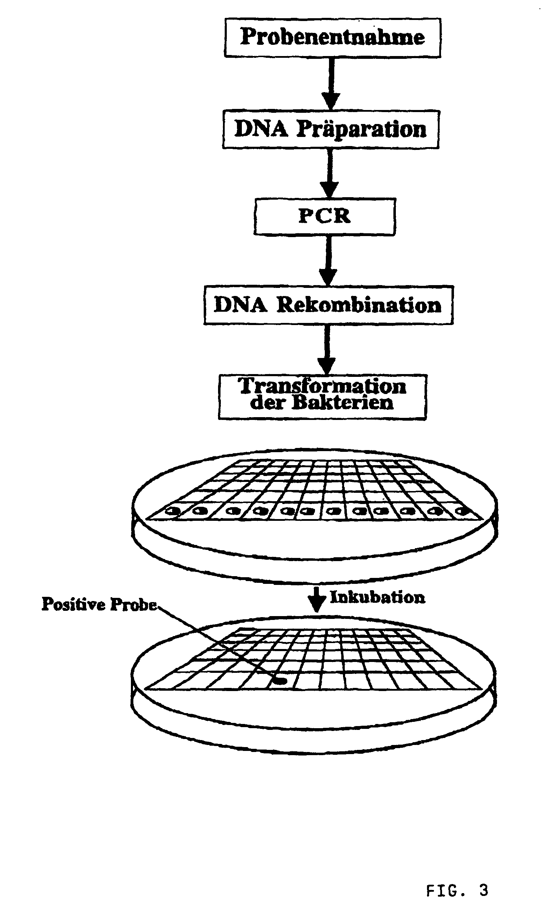

- Fig. 3: zeigt schematisch einen Workflow zur Identifizierung einer Mutation.

Im Sinne der Erfindung wird als nativer Leseraster der Leseraster einer Nucleotidsequenz verstanden, der die Synthese eines biologisch aktiven Genprodukts ermöglicht.

Weiterhin wird in der vorliegenden Erfindung der Begriff "Nucleotidsequenz, die für mindestens einen Teil eines Gens von Interesse codiert", eine der mRNA des Gens von Interesse entsprechende Nucleotidsequenz, sowie Teile davon verstanden bzw. auch von der genomischen Ebene abgeleitete Nucleotidsequenzen, die ein oder mehrere Exonsequenzen enthalten. Weiterhin kann sie von genomischer DNA aus dem Zellkern oder von mitochondrialer DNA abgeleitet sein.

Unter einer "Nucleotidsequenz, die den Leseraster aufrechterhält" wird eine Nucleotidsequenz mit einer Länge von etwa 3 bis zu etwa 300 Nucleotiden, vorzugsweise von etwa 30 bis etwa 210, mehr bevorzugt von etwa 70 bis etwa 210Nucleotiden verstanden, die im Leseraster mit der für das Toxin codierenden Nucleotidsequenz verknüpft ist, sowie im Leseraster mit der für mindestens einen Teil eines Gens von Interesse codierenden Nucleotidsequenz verknüpft ist. Bei letzterem wird die Verknüpfung im Leseraster als Verknüpfung mit dem nativen Leseraster verstanden.

Unter "Verknüpfung im Leseraster" wird im Sinne der Erfindung verstanden, dass eine Nucleotidsequenz mit einer anderen, ein Polypeptid codierenden Nucleotidsequenz ohne ein Stopcodon dazwischen verbunden wird, so dass bei Expression des Konstrukts ein Fusionsprotein gebildet werden kann. Bei der Nucleotidsequenz, die für mindestens einen Teil eines Gens von Interesse codiert, ist daher kein Stopcodon vorhanden, was durch Terminierung des Genfragments vor dem nativen Stopcodon oder durch Veränderung der Sequenz des Stopcodons erreicht werden kann.

Bei der Durchführung des Verfahrens ergeben sich nun 2 Möglichkeiten.

Ist das zu untersuchende Gen intakt, d.h. weist es keine Mutationen auf, die ein vorzeitiges Stopcodon in das Gen von Interesse einbringt, ggf. durch Punktmutation eines Nucleotids, oder die eine Änderung des Leserasters bewirken, wie beispielsweise durch Insertion oder Deletion von einem oder zwei Nucleotiden (oder mehrfachen davon, die kein neues Triplett ergeben), dann wird bei Exprimierung des Konstrukts ein Fusionspolypeptid synthetisiert, das die Aminosäuresequenz des Gens von Interesse, bzw. eines Teils davon, sowie C-terminal die Aminosäuresequenz des Toxins aufweist. Da das Toxin für die Wirtszelle schädlich ist, werden unter diesen Bedingungen alle dieses Konstrukt enthaltenden Zellen am Wachstum gehindert.

Enthält jedoch das zu untersuchende Gen eine der vorstehend genannten Mutationen, dann erfolgt die Expression eines Polypeptids, das kürzer ist als das Fusionspolypeptid. So wird beispielsweise bei Vorliegen eines vorzeitigen Stopcodons im Leseraster des nativen Gens das synthetisierte Produkt kürzer sein als das native Produkt, während bei Änderung des Leserasters, beispielsweise durch Insertion oder Deletion von 1, 2 bzw. 4 oder 5 usw.

Nucleotiden, der neue Leseraster bis zu einem Stopcodon darin weiterlaufen wird. Beiden Produkten gemeinsam ist das Fehlen eines aktiven, C-terminalen Toxin-Polypeptids im Fusionspolypeptid, da dieses entweder durch ein Stopcodon beendet wurde und somit die Proteinsequenz des Toxins nicht aufweist, oder das Toxin-Gen durch Änderung des Leserasters im Gen von Interesse in eine von dem Toxin-Gen abgeleite Proteinsequenz übersetzt wurde, die keine toxischen Auswirkungen auf die Zelle besitzt.

Somit führt im Falle des Vorliegens einer Mutation wie beschrieben, die Expression des Konstrukts zum Wachstum bzw. zur Bildung von Zellkolonien, so daß einfach durch das Erscheinen bzw. Wachsen von Zellkolonien ersichtlich ist, dass das Gen von Interesse durch eine Mutation wesentlich verändert wurde.

Ein derartiges Vorgehen ergibt gegenüber den bekannten Verfahren, insbesondere auch gegenüber der in der EP 0 872 560 offenbarten Methode den Vorteil eines unmittelbaren Ablesens des Ergebnisses am Wachstum der Zellen. Die Wahl eines Toxins, und insbesondere die dadurch durchgeführte "positive Selektion" auf Mutanten (bzw. negative Selektion auf Nicht-Mutanten), ergibt neben der Vereinfachung der Auswertung, auch den Vorteil, dass sich heterozygot vorliegende Mutationen eindeutig von den nicht wachsenden Zellen der Wildtyp-Gene unterscheiden lassen.

Eine Mutation kann auf diese Weise sogar noch in einem großen Überschuß an wildtyp Sequenzen erfasst werden. Weiterhin können die wachsenden Kolonien direkt als Quelle für ein amplifiziertes Genprodukt zur weiteren Charakterisierung der Mutation, beispielsweise durch Sequenzierung, verwendet werden. Dies ist auch bei einer Selektion von auxotrophen und prototrophen Mutanten nicht möglich, da dabei immer auf die "nicht-mutierten" Gene selektioniert wird.

Gemäß einer bevorzugten Ausführungsform ist die Nucleotidsequenz, die für mindestens einen Teil eines Gens von Interesse codiert, und das Toxin, durch eine Nucleotidsequenz getrennt, die den Leseraster aufrechterhält, und für eine Aminosäuresequenz codiert, die eine richtige Faltung des Toxins in Anwesenheit des Genprodukts des Gens von Interesse in dem Fusionsprotein ermöglicht.

Die vorliegende Erfindung basiert auf der Expression und der Wirkung des eingesetzten Toxins bei dem Vorliegen einer nativen Gensequenz. Da das Genprodukt der nativen Gensequenz aufgrund sterischer Gegebenheiten die Faltung des Toxin-Polypeptids negativ beeinflussen könnte, so dass dieses mindestens einen Teil seiner Toxizität für die Wirtszelle einbüssen kann, kann es bevorzugt sein, zwischen den das Gen von Interesse und das Toxin codierenden Nucleotidsequenzen eine Nucleotidsequenz vorzusehen, die Aminsoäuren codiert die als Abstandshalter zwischen den beiden Grundkörpern des Fusionspolypeptids agieren, so daß sich das Toxin trotz ggf. Vorhandensein eines sterisch ausufernden Genprodukts von Interesse dennoch richtig faltet und seine biologische Wirkung wahrnehmen kann. Eine derartige Nucleotidsequenz codiert vorzugsweise für Aminosäuren, die selbst keine sterische Behinderung darstellen, wie beispielsweise Glycin oder Alanin.

Gemäß einer weiteren bevorzugten Ausführungsform ist eine Nucleotidsequenz, die für mindestens einen Teil eines Gens von Interesse codiert, und die das Toxin codierende Nucleotidsequenz durch eine Nucleotidsequenz getrennt, die den Leseraster aufrechterhält und bei der in jedem Leseraster mit Maßgabe des ursprünglich richtigen Leserasters ein Stopcodon vorgesehen ist. Bei dieser Ausführungsform enthält die betreffende Nucleotidsequenz nur einen durchgehenden Leseraster, der mit dem des Gens von Interesse und dem des Toxins funktionell verbunden ist, d.h. bei Fehlen einer Mutation, die eine Veränderung des Leserasters in einen der anderen Leseraster bewirkt, ergibt das Konstrukt einen durchgehenden Leseraster der 3 Komponenten, das Gen von Interesse, die "Zwischen-Nucleotidsequenz" und das Toxin. Ist jedoch in der Nucleotidsequenz, die mindestens einen Teil eines Gens von Interesse codiert, aufgrund einer Mutation eine Änderung des Leserasters erfolgt, dann wird durch die "Zwischen-Nucleotidsequenz verhindert, dass die Nucleotidsequenz des Toxingens bzw. Teile davon exprimiert wird.

Das die verschiedenen, vorstehend aufgeführten Nucleotidsequenzen enthaltende Konstrukt weist zudem einen Promotor und ein Startcodon zur Expression des Fusionspolypeptids auf, sowie geeignete Sequenzen zur stabilen Weitergabe des Konstrukts an die Tochterzellen und ein Selektionsmarker. Der Promotor ist vorzugsweise induzierbar. Damit kann in einer ersten Ausführungsform durch Zugabe einer Substanz (Inducer) zum Kulturmedium der Promotor aktiviert und das Polypeptid exprimiert werden. Erfolgt eine derartige Induktion im wesentlichen unmittelbar nach der Transformation wird nur Zellen mit darin enthaltenen Konstrukten mit mutierten Nucleotidsequenzen von Interesse ein Wachstum ermöglicht. Wird der Promotor jedoch nicht aktiviert, dann werden alle ein Konstrukt enthaltenen Zellen wachsen. Die Kolonienzahl in diesem Fall kann damit als Kontrollwert für die Gesamtzzahl der Zellen die ein Konstrukt enthalten dienen, unabhängig ob von einem wildtyp- oder mutierten Gen.

Als Toxin kann jedes für die entsprechende Wirtszelle geeignete bzw. bekannte toxische Genprodukt eingesetzt werden, wie beispielsweise das ccdB Gen des F Plasmids, das GenE des Phagen ΦX174, oder Colicin für die Anwendung in Bakterien.

Als die Wirtszelle zur Expression des Konstrukts kann jede geeignete Zelle eingesetzt werden, in der das Konstrukt zur Expression gebracht werden kann. Beispiele geeigneter Wirtszellen sind Bakterienzellen, wie E.coli, Hefezellen, Insektenzellen oder Säugerzellen, bzw. davon abgeleitete Zelllinien. Gemäß einer bevorzugten Ausführungsform werden E. coli verwendet, die ein schnelles und verläßliches Arbeiten ermöglichen.

Das Verfahren eignet sich in hervorragendem Maße zur Durchmusterung grösserer Populationen, wie Tierpopulationen, beispielsweise Rinder, Schweine, Ziegen oder Schafe. Die Tiere können damit schnell auf das Vorhandensein einer Mutation untersucht werden, die in den jeweiligen Tieren zwar rezessiv und damit phänotypisch nicht erkennbar sind, jedoch bei Kreuzung mit einem diese Mutation ebenfalls tragenden Tier, Nachkommen hervorbringen können, bei denen die Mutation phänotypisch zum Tragen kommt.

Das Verfahren ist zudem zum Nachweis der Neigung eines Individuums, beispielsweise eines Tiers, insbesondere eines Menschen, zur Entwicklung einer Erkrankung von Nutzen.

Bei der Krebsentstehung spielen bekanntermaßen Tumorsuppressorgene eine wichtige Rolle. Sie schützen die Zellen vor unkontrolliertem Wachstum. Wird ein Allel des Gens inaktiviert so ist noch ein zweites vorhanden, das die Funktion erfüllen kann. Erst ein weiteres Mutationsereignis im selben Genlokus führt zur kompletten Ausschaltung dieser wichtigen Kontrollfunktion. Die erste Mutation kann lange vor Ausbruch der Krankheit stattgefunden haben und unerkannt bleiben oder von Eltern vererbt worden sein. Die vorliegende Erfindung liefert daher ein schnelles und zuverlässiges Verfahren zur Bestimmung der Neigung eines Individuums zur Entwicklung von Krebs, wobei Tumorsuppressorgene in einem Individuum auf deren Funktionsfähigkeit untersucht werden.

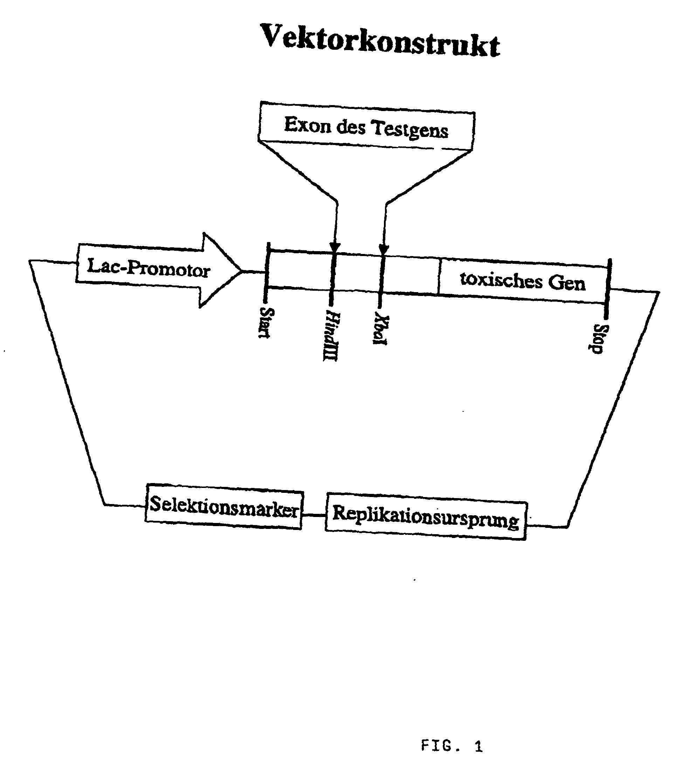

Grundlage der Methode ist die Überprüfung des offenen Leserasters des Gens in einem in vivo Test in Wirtszellen, wie den vorstehend erwähnten. (siehe Fig. 1).

Die Nucleotidsequenz eines Gens von Interesse kann gemäß allen bekannten Verfahren erhalten werden, wie beispielsweise durch Herstellung von cDNA. Zudem kann die Methode der PCR eingesetzt werden, wobei die damit hergestellte cDNA oder ein Exon, d.h. ein durchgehendes Stück des Leserasters aus genomischer DNA des Individuums amplifiziert und dann in einen Vektor vor das Toxin eingebaut wird. Ist der Leseraster korrekt und enthält keine Stopcodons, so wird das Toxin translatiert und führt zum Absterben der Wirtszelle.

Auch der Einsatz mehrerer Genfragmente, unabhängig davon ob sie vom gleichen oder von verschiedenen Genen stammen, in einer Analysenreaktion ist möglich. In einer bevorzugten Ausführungsform werden daher mittels Multiplex-PCR mehrere Genfragmente gleichzeitig amplifiziert und in der Folge auf Mutationen analysiert.

Bei der PCR sollten die Primer am 5' Ende lange Überhänge für die DNA Rekombination aufweisen. Damit bei diesem Aufbau keine zu geringen Ausbeuten und unerwünschte Nebenprodukte erhalten werden, kann eine sogenannte zweistufige, "nested PCR" gewählt werden. Bei dieser Methode wird eine erste PCR Stufe mit äußeren Primern durchgeführt und das Produkt in einer zweiten Stufe mit inneren Primern noch einmal selektiv amplifiziert. Um dabei einen schnellen Probendurchsatz zu ermöglichen, wird der PCR-Ansatz in einem Behälter ohne Aufreinigung vor Zugabe der inneren Primer durchgeführt. Dazu werden die äußeren Primer für die erste Reaktion in einer Länge von ca. 12 ca. 16 Nucleotiden gewählt was eine Annealingtemperatur von ca. ≤ 40 °C erfordert. Für Amplifizierung des Produkts mittels der innen liegenden Primer werden diese länger gewählt, d.h. mit einer Länge und/oder Nudeotidzusanunensetzung, die eine Annealingtemperatur von oberhalb der Temperatur der ersten Stufe erfordert (z.B. etwa 50 °C). Bei dieser Vorgehensweise kann ein gewünschtes Produkt schnell und hochspezifisch erhalten werden.

Das so erhalten Fragment wird dann in einen geeigneten Vektor inseriert, wobei jedes bekannte Verfahren eingesetzt werden kann. Gemäß einer bevorzugten Ausführungsform wird die sogenannte UDG-Methode eingesetzt (siehe WO 92/22649). Dabei werden PCR Primer mit Uracil anstatt Thymin verwendet. Das Enzym Uracil-DNA-Glycosylase (UDG) schneidet in der Folge in den PCR Produkten das Uracil im Primerbereich heraus, wodurch in diesem Bereich nur der Gegenstrang in Form einzelsträngiger DNA übrig bleibt. Eine Inkubation bei 37° mit einem in gleicher Weise behandelten Vektor ermöglicht dann eine Anlagerung der komplementären einzelsträngigen DNA-Bereiche. Nach Transformation in E. coli werden die Lücken in der DNA durch das Reparatursystem geschlossen, wodurch funktionsfähige ringförmige Plasmide entstehen.

Nach Inserieren des Fragments in einen Vektor werden die rekombinierten DNA Moleküle in Bakterienzellen transformiert. Dabei kann ebenfalls jede bekannte und geeignete Transformationsmethode eingesetzt werden, wie sie beispielsweise in Maniats et al., A Laboratory Handbook (1992) beschrieben sind.

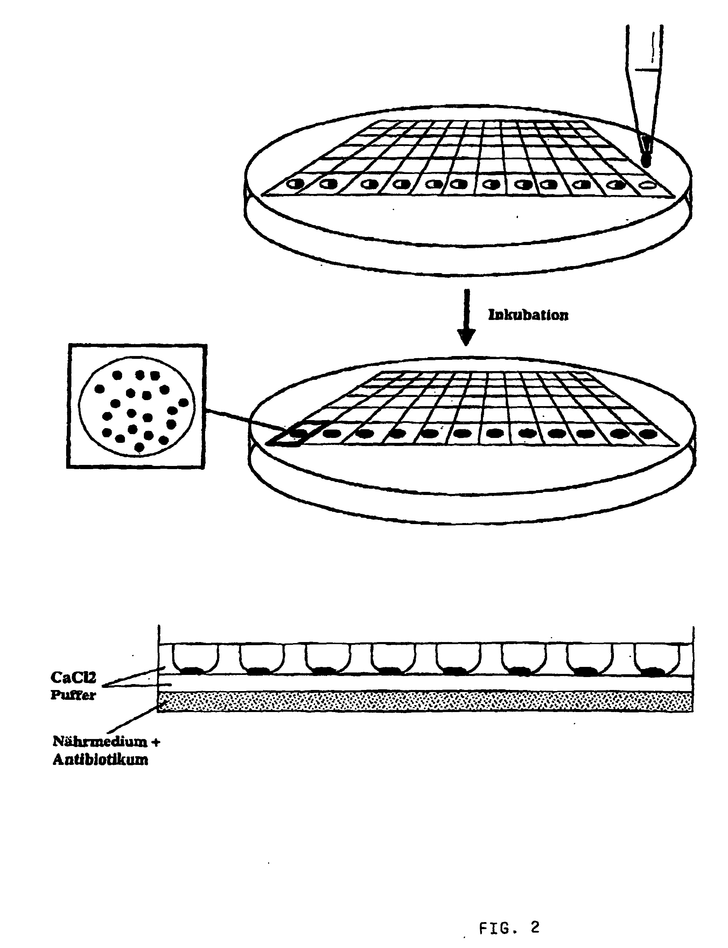

Gemäß einer bevorzugten Ausführungsform wir ein Verfahren eingesetzt, bei dem die Transformation direkt auf der Platte erfolgt. Dabei kommen die DNA und die kompetenten Bakterien erst auf dem Nährmedium miteinander in Kontakt. Da Bakterien jedoch bei Kontakt mit Nährmedium ihre Fähigkeit DNA aufzunehmen verlieren wird ein mehrschichtiger Aufbau der Platten (siehe Fig. 2) verwendet.

Die oberste Schicht besteht aus einem CaCl₂-haltigen Puffer, der die Zellen kompetent hält. Die darunterliegende Schicht enthält Nährmedium und Antibiotikum. Während der Transformation sind daher optimale Bedingungen für die Aufnahme der DNA gegeben, danach gelangen die Nährstoffe und das Antibiotikum durch Diffusion zu den Bakterienzellen. Durch Ausbildung von Vertiefungen können mit dieser Methode viele Proben gleichzeitig auf einer Platte transformiert werden, wobei die Schritte mit standardisiertem 96-well Format kompatibel werden.

Die Erfindung wird durch die folgenden Beispiele weiter erläutert, die jedoch nicht als einschränkend angesehen werden sollen.

### Beispiel 1

Das Analyseschema wurde anhand des α-1,3-Galactosyltransferasegens beim Schwein ausgearbeitet. Ziel ist es hier ein Schwein zu identifizieren, bei dem die enzymatische Aktivität dieses Gens inaktiviert ist, was für eine Anwendung bei Xenotransplantationen von großer Bedeutung wäre. Das relativ große Exon 9 beinhaltet etwa 2/3 der kodierenden Region dieses Gens, während das restliche Drittel auf 4 kleine Exons aufgesplittert ist. Allerdings befindet sich dieses große Exon am Ende der kodierenden Region. Es konnte jedoch gezeigt werden, daß bereits die Deletion der letzten drei Aminosäuren zum kompletten Verlust der enzymatischen Aktivität führt (Henion et. al., Glycobiology 4 (1994), 193). Aus diesem Grunde wurde dieses Exon für die Analyse ausgewählt.
1. Die Präparation der genomischen DNA erfolgte von Gewebsproben gemäß herkömmlicher Methoden.
2. Mittels zweistufiger PCR wurde ein 729 bp großes Fragment vom Exon 9 des α-1,3-Galaktosyltransferasegens amplifiziert. In der ersten Stufe wurde das Primerpaar 1 (Oligonukleotide 1 und 2; siehe Tabelle 1) verwendet. Die Reaktion erfolgte in einem Volumen von 20 µl mit 10 ng genomischer DNA (Reaktionsbedingungen: 10 mM Tris pH 9,2; 50 mM KCl; 1 U Taq Polymerase; je 0,1 µM Primer; 200 µM je dNTP; Temperaturprofil: 5 min bei 94°, dann 15 Zyklen mit 20 s bei 94°, 30 s bei 38° und 1 min bei 72°). Dann wurde 1 µl mit je 8 pmol an Oligonukleotiden 3 und 4 zugegeben und die Reaktion für weitere 20 Zyklen (Temperaturprofil: 20 s bei 94°, 30 s bei 54° und 1 min bei 72°, sowie am Schluß der Reaktion eine weitere Inkubation von 5 min bei 72°) inkubiert.
3. Zur Herstellung des Konstrukts wurde das Plasmid pZERO-2 (INVITROGEN / USA) adaptiert, das ein toxisches Gen (*ccd*B) unter Kontrolle eines induzierbaren Lac Promotors enthält. Die Kanamycin Resistenz des Plasmids pZERO-2 war jedoch für die nachfolgende Transformation ungünstig und wurde daher in eine Ampicillin Resistenz umgewandelt. Dazu wurde die Region des Lac Promotors und des *ccd*B Gens mit Hilfe der Restriktionsenzyme *Afl*III (LIFE TECHNOLOGIES / Österreich) und *Eco147*I (FERMENTAS / Deutschland) herausgeschnitten und in das Plasmid pUC 19 (geschnitten mit *Afl*III und *Aat*II (FERMENTAS / Deutschland) und danach behandelt mit T4 DNA Polymerase (FERMENTAS / Deutschland) ligiert (T4 DNA Ligase FERMENTAS / Deutschland, alle Reaktionen wurden entsprechend den Firmenangaben bzw. molekularbiologischen Standardmethoden durchgeführt), was in dem Plasmid pZ resultierte. Anders als bei der beschriebenen Präparation des Vektors für die UDG-Klonierung (Rashtchian et. al. Analytical Biochemistry **206** (1992), 91)) etablierten wir eine vereinfachte Methode, die aber zu einer deutlichen Reduktion unerwünschter Nebenprodukte führte. Statt zwei komplementäre, Uracil enthaltende Oligonukleotide an die Enden des geschnittenen Vektors zu ligieren, verwendeten wir nur ein phosphoryliertes Oligonukleotid ohne Uracil, das direkt in den DNA Überhang des Restriktionsenzyms ligiert wurde. Dadurch entstehen von vorneherein genau definierte einzelsträngige Bereiche an den Vektorenden. Zur Positionierung der Schnittstellen im Leserasters des Polylinkers wurde das Plasmid pZ mit den Enzymen *Hind*III (LIFE TECHNOLOGIES / Österreich) und *Xba*I (LIFE TECHNOLOGIES /Österreich) geschnitten und mit den phosphorylierten Oligonukleotiden 5 und 6 ligiert, wobei das Plasmid pZ2 erhalten wurde. Für die DNA-Rekombination wurde dieses Plasmid folgendermaßen präpariert: 5 µg des Plasmids wurden mit je 40 U *Hind*III (LIFE TECHNOLOGIES / Österreich) und *Xba*I nach Herstellerangaben verdaut. Danach wurde mit 10 U Shrimp Alkaline Phosphatase (FERMENTAS /Deutschland) für 1 Stunde bei 37° inkubiert und in weiterer Folge wurde das Enzym durch eine 20 minütige Inkubation bei 65° inaktiviert. Nach Zugabe von jeweils 130 pmol phosphorylierter Oligonukleotide 7 und 8 wurde bei 14° über Nacht in einem Volumen von 100 µl mit 20 U T4 DNA Ligase (FERMENTAS / Deutschland), 30 U *Xba*I und Ligasepuffer laut Herstellerangaben inkubiert. Nach 10 minütigem Aufheizen auf 65° wurde die DNA über ein 1% Agarosegel gereinigt (mit einem Cleanmix Kit TALENT/Italien) und quantifiziert. Für die eigentliche Rekombinierungsreaktion wurden 2 ng dieses Vektors zusammen mit 1 µl der vorstehend beschriebenen PCR Reaktion, 1 µl eines Puffers (10 mM Tris pH 8.3; 50 mM KCl; 1.5 mM MgCl2), sowie 0,1 U Uracil DNA Glycosylase (LIFE TECHNOLOGIES /Österreich) und Wasser in einem Gesamtvolumen von 12,5 µl für 90 min bei 37° inkubiert.
4. Die Platten für die Transformation wurden folgendermaßen präpariert: In 15 cm Petrischalen (GREINER/Österreich) wurde eine erste Agaroseschicht gegossen, die folgendermaßen hergestellt wurde: 25 ml autoklaviertes LB-Medium, das 0,5 g Select Pepton 140 (LIFE TECHNOLOGIES / Österreich); 0,25 g Hefeextrakt für die Bakteriologie (ROTH / Deutschland); 0,25 g NaCl (FLUKA / Österreich); 50 µl 1 M NaOH (FLUKA / Österreich) enthält wurde mit 0,375 g Agarose Electrophoresis Grade (LIFE TECHNOLOGIES / Österreich) aufgekocht und dann auf 60° temperiert. Nach Zugabe von 10 mg Ampicillin (SIGMA / Österreich) wurden die Platten gegossen, nach dem Erstarren für 90 Minuten unter sterilem Luftstrom getrocknet und dann für 1 bis 3 Tage bei 4° aufbewahrt. Die gekühlten Platten wurden dann für 1 Stunde bei 20° temperiert und dann die zweite Agaroseschicht gegossen, die folgendermaßen hergestellt wurde: 40 ml autoklavierter CaCl₂ Puffer, der 0,36 g CaCl₂.H₂O (MERCK / Österreich) und 0,084 g 3-Morpholinopropansulfonsäure (MERCK / Österreich) bei einem pH-Wert von 5,5 enthält wurden mit 0,6 g Agarose aufgekocht, dann bei 56° temperiert, wenn notwendig 40 µl einer 2 M sterilfiltrierten Lösung IPTG (Isopropyl-β-D-Thiogalactopyranoside, BTS/Deutschland) zugegeben und dann auf die erste Agaroseschicht gegossen. Nach 10 Minuten auf Raumtemperatur wurden die Platten für 40 min im sterilen Luftstrom getrocknet und dann 30 Minuten im Kühlraum auf Eis abgekühlt. In weiterer Folge wurde eine dritte Agaroseschicht von 35 ml gegossen, die in gleicher Weise hergestellt wurde wie die zweite Schicht, allerdings wurde nach dem Gießen sofort eine 96-well formatige Platte mit halbrunden Ausbuchtungen in die Agaroseschicht gedrückt. Nach 10 Minuten wurde die Plastikform von der erstarrten Agaroseschicht gelöst und die Platten wieder auf Eis im Kühlraum inkubiert. Nach 2 Stunden wurden je Vertiefung 2 µl kompetente Bakterien (siehe unten) und 2 µl der vorstehend beschriebenen UDG-Reaktion pipettiert. Nach einer weiteren Inkubation von 30 Minuten auf Eis und einer Stunde bei Raumtemperatur wurden die Platten nicht invertiert bei 37° für etwa 14 Stunden inkubiert.

Für die Präparation der kompetenten E. coli Bakterien wurde folgendes Protokoll verwendet: Eine Einzelkolonie XL10-Gold (STRATAGENE / Deutschland) diente zum Animpfen einer 50 ml Kultur mit LB-Medium (siehe oben). Nach einer etwa 14 stündigen Inkubation im Schüttler bei 37° wurden 4 ml zum Beimpfen einer 400 ml Kultur (LB-Medium) verwendet. Bei einer optischen Dichte von 0,3 bei 600 nm wurden die Bakterien für 5 Minuten auf Eis inkubiert und dann in einer Kühlzentrifuge (J6-MI BECKMAN/USA; Rotor JS-4.2) bei 4° mit 1600 Umdrehungen für 7 Minuten zentrifugiert. Der Überstand wurde abgesaugt und das Pellet in 10 ml eisgekültem CaCl₂ Puffer suspendiert. Dieser Puffer enthält pro Liter 8,82 g CaCl₂.H₂O (MERCK / Österreich) 150 g Glycerin wasserfrei reinst (MERCK / Österreich) und 2,1 g 3-Morpholinopropansulfonsäure (MERCK / Österreich) bei einem pH-Wert von 7,0 und wird vor der Verwendung autoklaviert. Diese Lösung wird für 5 Minuten bei 1100 Umdrehungen zentrifugiert (Rest wie oben), das Pellet wieder in 10 ml CaCl₂ Puffer suspendiert und 30 Minuten auf Eis inkubiert. Nach einem weiteren Zentrifugationsschritt (wie vorher) wird das Pellet in 2 ml CaCl₂ Puffer suspendiert und in Aliquots bei -80° gelagert. Für die vorstehend beschriebene Transformation auf Platte wurden diese kompetenten Bakterien direkt vor dem Versuch 1:9 mit eiskaltem CaCl₂ Puffer verdünnt.

Die vorstehend beschriebene UDG-Reaktion wurde auf zwei verschiedene Platten transformiert: Eine mit, und eine ohne IPTG (siehe oben). In Anwesenheit von IPTG wird der Lac-Promotor des Vektorkonstrukts aktiviert und in der Folge kann das toxische *ccd*B Gen exprimiert werden. Nur auf diesen selektiven Platten sind daher Mutationen an erhöhter Kolonienzahl erkennbar. Die nicht-selektive Platte ohne IPTG stellt jedoch eine Kontrolle dar ob die DNA Rekombination überhaupt funktioniert hat. Als Ergebnis wurde die Anzahl der Kolonien auf der selektiven Platte in % von der Kolonienzahl der nicht selektiven Platte gewertet. Als Positivkontrolle verwendeten wir ein Fragment vom Exon 9 der Galaktosyltransferase, das wir durch Verdau mit den Restriktionsenzymen *Bpu1102*I und *Eco147*I, sowie Auffüllen der Enden mit dem Klenow Fragment der DNA-Polymerase und Religation mit T4 DNA Ligase (alle Enzyme FERMENTAS/Deutschland; Reaktionsbedingungen wie vom Hersteller angegeben) *in vitro* mutiert hatten. Diese Mutation führt zu einer 8 bp langen Deletion die daher in einer Verschiebung des Leserasters resultiert. 1 pg dieses Fragments wurde statt genomischer DNA in der PCR eingesetzt. Um eine heterozygote Situation zu simulieren mischten wir je 0,5 pg eines mutierten und eines wildtyp Fragments.

Bei einem typischen Experiment mit DNA Proben von 84 Schweinen wurden folgende Resultate gefunden: 82 Proben ergaben zwischen 0 und 15 % an Kolonien auf der selektiven Platte mit einem Durchschnittswert von 5,9 %, zwei Proben zeigten weniger als 5 Kolonien auf der nicht-selektiven Platte und wurden daher nicht gewertet. Die Kolonien auf der selektiven Platte sind nicht auf Mutationen, sondern auf Nebenprodukte der DNA-Rekombination, sowie Fehler der Taq Polymerase zurückzuführen, wie eine genauere Analyse mittels Sequenzieren zeigte. 4 Positivkontrollen (homozygot) ergaben 80 - 105 % (Mittelwert 97%) und 4 heterozygote Positivkontrollen 41 - 98 % (Mittelwert 71%). Unerwarteter Weise lag der Wert für die heterozygote Positivkontrolle deutlich über 50 %. Auch bei anderen Experimenten erhielten wir im Durchschnitt Werte über 50 %, was wahrscheinlich wieder auf Nebenreaktionen zurückzuführen ist. Damit ergibt sich ein ausreichender Spielraum um heterozygote Mutanten von wildtyp Tieren zu unterscheiden. Zusammenfassend zeigten diese Experimente mit der Galaktosyltransferase eine hohe Verläßlichkeit der Methode.

### Beispiel 2

### Analyse von Rindern die Stopcodonmutationen aufweisen

Verschiedene Mutationen natürlichen Ursprungs sind bisher bei Rindern beschrieben. So ist eine Mutation im Uridinmonophosphatsynthasegen beschrieben (DUMPS, deficiency of uridine monophosphate synthase) sowie eine Mutation die zu Citrullinämie führt. Während diese Mutationen unerwünscht sind, da sie bei homzygoten Individuen zu gefährlichen Stoffwechselstörungen führen, geht eine Mutation im Myostatingen mit einer dramatischen Zunahme der Muskelmasse einher, was durchaus ein erwünschtes Zuchtziel darstellt. Allen diesen natürlichen Mutationen ist gemeinsam, daß sie zu vorzeitigem Abbruch des Leserasters führen. Eine dieser Mutationen wurde im Uridinmonophosphatsynthasegen als Beispiel genommen um diese Methode auszutesten. Die Ergebnisse sind in nachstehender Tabelle 1 aufgezeigt:

**Tabelle 1**

| | Nummer | % |
|---|---|---|
| wildtyp | 1 | ∼5 |
| | 2 | ∼5 |
| | 3 | ∼5 |
| Heterozygot | 4 | ∼55 |
| | 5 | ∼55 |
| | 6 | ∼55 |

### Beispiel 3

### Analyse von Zebrafischen die Stopcodonmutationen aufweisen

Durch eine kurze Generationszeit und geringen Platzbedarf eignen sich einige Fischarten besonders gut für genetische Experimente. Durch Exposition mit der mutagenen Substanz Ethylnitrosurea (ENU) werden mit hoher Effizienz Punktmutationen im Genom induziert. In mehreren genetischen Screens konnten auf diese Weise bei Zebrabärblingen hunderte von Mutanten generiert werden. In einigen Fällen konnten die zugehörigen Gene identifiziert werden. Ein Beispieldafür ist die Bozozok Mutante. Beim Allel *boz*^{*m168*} wird durch einen Basenaustausch ein vorzeitiges Stopcodon generiert wodurch die Funktion des Gens inaktiviert wird (Fekany et al., Development 126 (1999), 1427). Dieses Allel wurde verwendet, um am Beispiel einer induzierten Mutation die erfindungsgemäße Methode zu testen, wobei der Versuchsaufbau gemäß Beispiel 1 verwendet wurde. Die Ergebnisse sind in folgender Tabelle 2 gezeigt:

**Tabelle 2**

| | Nummer | % |
|---|---|---|
| wildtyp | 1 | 9 |
| | 2 | 10 |
| | 3 | 10 |
| heterozygot | 4 | 58 |
| | 5 | 88 |
| | 6 | 57 |
| homozygot | 7 | 75 |
| | 8 | 100 |
| | 9 | 116 |

Zusätzlich wurde mit diesen DNA Proben auch die Sensitivität des Verfahrens geprüft. Dazu wurden in einem weiteren Experiment nach der PCR die Fragmente aus einem Agarosegel ausgeschnitten, gereinigt (mit einem Cleanmix Kit TALENT/Italien) und quantifiziert. Danach wurden die Fragmente von wildtyp DNA mit geringen Mengen an Fragmenten von heterozygoten Mutanten gemischt. Die Ergebnisse sind in Tabelle 3 gezeigt:

**Tabelle 3**

| | Nummer | % |
|---|---|---|
| 100% wildtyp | 1 | 2 |
| | 2 | 1 |
| | 3 | 2 |
| 80% wildtyp + 20% heterozygot | 4 | 15 |
| | 5 | 16 |
| | 6 | 29 |
| 90% wildtyp + 10% heterozygot | 7 | 5 |
| | 8 | 8 |
| | 9 | 5,5 |

Die gereinigten Fragmente zeigten in diesem Experiment eine deutlich verringerte Kolonienzahl auf den selektiven IPTG hältigen Platten (Durchschnitt 2%). Bei einem fünffachen Überschuß an DNA von heterozygoten Fischen (entspricht einem zehnfachen Überschuß an wildtyp versus mutiertem Allel) war ein deutlicher Unterschied zu den reinen wildtyp Proben zu sehen (Durchschnitt 19%). Sogar bei einer weiteren Verschiebung des Verhältnisses zu einem zehnfachen Überschuß an heterozygoter DNA (entspricht einem zwanzigfachen Überschuß an wilttyp versus mutiertem Allel) war noch ein geringer Unterschied in der Kolonienzahl zu sehen (Durchschnitt 6%). Diese Experimente zeigen, daß das Verfahren hochsensitiv ist und auch bei gemischen Proben noch mutierte Allele detektieren kann.

## Patentansprüche

1. Verfahren zum Nachweis von Mutationen in einem Gen, bei denen der native Leseraster zerstört ist, welches umfaßt,
(a) Herstellen eines Konstrukts umfassend eine Nucleotidsequenz, die für mindestens einen Teil eines Gens von Interesse codiert und eine ein Toxin codierende Nucleotidsequenz, die stromabwärts der Nucleotidsequenz, die für mindestens einen Teil eines Gens von Interesse codiert, mit dieser im Leseraster davon verknüpft ist, wobei das Toxin für die Wirtszelle schädlich ist,
(b) Exprimieren des Konstrukts in einer geeigneten Wirtszelle, wobei
das Wachstum der bakteriellen Wirtszelle ein Anzeichen für eine in dem Gen von Interesse enthaltene Mutation ist.

2. Verfahren nach Anspruch 1, wobei die Nucleotidsequenz, die für mindestens einen Teil eines Gens von Interesse codiert, und das Toxin durch eine Nucleotidsequenz getrennt sind, die den Leseraster aufrechterhält, und für eine Aminosäuresequenz codiert, die eine richtige Faltung des Toxins in Anwesenheit des Genprodukts des Gens von Interesse in dem Fusionsprotein unterstützt.

3. Verfahren nach Anspruch 1, wobei die Nucleotidsequenz, die für mindestens einen Teil eines Gens von Interesse codiert, und die das Toxin codierende Nucleotidsequenz durch eine Nucleotidsequenz getrennt sind, die den Leseraster aufrechterhält und bei der in jedem Leseraster mit Maßgabe des ursprünglich richtigen Leserasters ein Stopcodon vorgesehen ist.

4. Verfahren nach Anspruch 1, wobei das Toxin das ecdB Gen des F Plasmids, das Gen E des Phagen ΦX174, oder Colicin ist.

5. Verfahren nach einem der vorhergehenden Ansprüche, wobei die Wirtszelle eine bakterielle Zelle ist, eine Hefezelle, eine Insektenzelle oder eine Säugerzelle.

6. Verfahren nach einem der vorhergehenden Ansprüche, wobei das Verfahren bei der Durchmusterung von Tierpopulationen eingesetzt wird.

7. Verfahren nach Anspruch 6, wobei die Tierpopulation Rinder, Schweine, Ziegen oder Schafe sind.

8. Verfahren nach einem der Ansprüche 1 bis 5, zur Diagnostik der Neigung zur Entwicklung einer Erkrankung in einem Individuum.

9. Verfahren nach Anspruch 8, wobei die Erkrankung Krebs ist.

10. Verfahren nach Anspruch 9, wobei das Gen von Interesse ein Tumorsupressorgen ist.

11. Verfahren nach einem der Ansprüche 1 bis 5, wobei das Verfahren zur Durchmusterung einer Gruppe von künstlich mutierten Individuen eingesetzt wird.

## Claims

1. A method for detecting mutations in a gene in which the native reading frame has been destroyed, comprising,
a) Preparing a construct comprising a nucleotide sequence which encodes for at least one part of a gene of interest and a toxin encoding nucleotide sequence which downstream from the nucleotide sequence which encodes for at least one part of a gene of interest is linked to this in the reading frame thereof, wherein said toxin is harmful for the host cell,
b) Expressing said construct in a suitable host cell, wherein the growth of said bacterial host cell is an indication for a mutation contained in said gene of interest.

2. The method according to claim 1, wherein said nucleotide sequence, which encodes for at least one part of a gene of interest and the toxin are separated by a nucleotide sequence which maintains said reading frame and encodes for an amino acid sequence which supports a proper folding of said toxin in the presence of the gene product of said gene of interest in the fusion protein.

3. The method according to claim 1, wherein said nucleotide sequence which encodes for at least one part of a gene of interest and said nucleotide sequence encoding for said toxin are separated by a nucleotide sequence which maintains said reading frame and in which in each reading frame with the proviso of said original correct reading frame a stop codon is provided.

4. The method according to claim 1, wherein said toxin is the ccdB gene of the F plasmid, the gene E of the phage ΦX174 or colicin.

5. The method according to any of the preceding claims, wherein said host cell is a bacterial cell, a yeast cell, an insect cell or a mammalian cell.

6. The method according to any of the preceding claims, wherein said method is employed in the screening of animal populations.

7. The method according to claim 6, wherein the animal population are bovine, pigs, goats or sheep.

8. The method according to any of the claims 1 to 5 for diagnosis of the predisposition for the development of a disease in an individual.

9. The method according to claim 8, wherein the disease is cancer.

10. The method according to claim 9, wherein said gene of interest is a tumor suppressor gene.

11. The method according to any of the claims 1 to 5, wherein said method is employed for screening a group of artificially mutated individuals.

## Revendications

1. Procédé pour la détection de mutations au sein d'un gène dans lequel le cadre de lecture natif est détruit, lequel comprend
a) la production d'une construction comprenant une séquence de nucléotides codant pour au moins une portion d'un gène d'intérêt et une séquence de nucléotides codant pour une une toxine qui, en aval de la séquence de nucléotides codant pour au moins une portion d'un gène d'intérêt, est connectée avec cette dernière dans le cadre de lecture de celle-ci, dans laquelle la toxine est nocive pour la cellule hôte,
b) l'expression de la construction dans une cellule hôte appropriée, dans laquelle la croissance de la cellule hôte bactérienne est un indice pour une mutation au sein du gène d'intérêt.

2. Procédé selon la revendication 1, dans lequel la séquence de nucléotides codant pour au moins une portion d'un gène d'intérêt et la toxine sont séparées par une séquence de nucléotides qui supporte le cadre de lecture et code pour une séquence d'acides aminés qui assiste le pliage correct de la toxine en absence du produit génique du gène d'intérêt dans la protéine de fusion.

3. Procédé selon la revendication 1, dans lequel la séquence de nucléotides codant pour au moins une portion d'un gène d'intérêt et la séquence de nucléotides codant pour la toxine sont séparées par une séquence de nucléotides qui supporte le cadre de lecture et par laquelle est pourvu, dans chaque cadre de lecture, un codon stoppeur avec l'instruction du cadre de lecture original approprié.

4. Procédé selon la revendication 1, dans lequel la toxine est le gène ccdB du plasmide F, le gène E du phage ΦX174 ou la colicine.

5. Procédé selon l'une des revendications précédentes, dans lequel la cellule hôte est une cellule bactérienne, une cellule de levure, une cellule d'insecte ou une cellule de mammifère.

6. Procédé selon l'une des revendications précédentes, dans lequel le procédé est mis en oeuvre pour l'échantillonnage d'une population animale.

7. Procédé selon la revendication 6, dans lequel la population animale comprend les bovins, les porcs, les chèvres ou les moutons.

8. Procédé selon l'une des revendications 1 à 5 pour le diagnostic de la prédisposition au développement d'une maladie chez un individu.

9. Procédé selon la revendication 8, dans lequel la maladie est le cancer.

10. Procédé selon la revendication 9, dans lequel le gène d'intérêt en un gène suppresseur de tumeur.

11. Procédé selon l'une des revendications 1 à 5, dans lequel le procédé est mis en oeuvre pour l'échantillonnage d'un groupe d'individus artificiellement mutés.
